# EUROPÄISCHE PATENTSCHRIFT

(11) **EP 4 398 799 B1**
(45) Veröffentlichungstag und Bekanntmachung des Hinweises auf die Patenterteilung: **25.03.2026**
(21) Anmeldenummer: 22772921.7
(22) Anmeldetag: 01.09.2022
(51) Int. Cl.: A61B 5/243, A61B 5/00

(54) **VERFAHREN UND EINRICHTUNG ZUR BESTIMMUNG DER VITALFUNKTIONEN EINES FAHRZEUGINSASSEN**
METHOD AND DEVICE FOR DETERMINING THE VITAL FUNCTIONS OF A VEHICLE OCCUPANT
PROCÉDÉ ET DISPOSITIF PERMETTANT DE DÉTERMINER LES FONCTIONS VITALES D'UN OCCUPANT D'UN VÉHICULE

(30) Priorität: 06.09.2021 DE 102021209759
(43) Veröffentlichungstag der Anmeldung: 17.07.2024
(73) Patentinhaber: Robert Bosch GmbH, 70442 Stuttgart (DE)
(72) Erfinder: FUCHS, Tino, 72076 Tuebingen (DE); BIEG, Hans-Joachim, 71093 Weil Im Schoenbuch (DE); ROELVER, Robert, 75365 Calw-Stammheim (DE)
(86) Internationale Anmeldenummer: PCT/EP2022/074353
(87) Internationale Veröffentlichungsnummer: WO 2023/031339

(56) Entgegenhaltungen:
- US-A1- 2009 326 399
- KEIGO ARAI ET AL: "Millimetre-scale magnetocardiography of living rats using a solid-state quantum sensor", ARXIV.ORG, CORNELL UNIVERSITY LIBRARY, 201 OLIN LIBRARY CORNELL UNIVERSITY ITHACA, NY 14853, 25 May 2021 (2021-05-25), XP081968778

## Beschreibung

Die vorliegende Beschreibung betrifft ein Verfahren und eine Einrichtung zur Bestimmung der Vitalfunktionen eines Fahrzeuginsassen, wobei von einer in einen Fahrzeugsitz eines Kraftfahrzeugs integrierten Sensorvorrichtung berührungslos Kardiogrammsignale eines Fahrzeuginsassen ermittelt werden, wobei die Kardiogrammsignale an eine Auswerteeinheit übermittelt werden, wobei die Auswerteeinheit aus den Kardiogrammsignalen Vitalfunktionen des Fahrzeuginsassen ermittelt.

Ferner betrifft die vorliegende Beschreibung ein Kraftfahrzeug umfassend einen Fahrzeugsitz und eine Auswerteeinheit.

### Stand der Technik

Im Bereich der Entwicklung von Mobilitätslösungen gewinnen die Bereiche autonomes Fahren, Gesundheit und Passagiersicherheit zunehmend an Bedeutung.

Die Erkennung des Verhaltens eines Fahrzeugführers sowie der weiteren Fahrzeuginsassen ist heute schon integraler Bestandteil von Kraftfahrzeugen. So sind beispielsweise die Erkennung der Sitzbelegung in Kombination mit dem Anschnallsensor oder eine Fahrermüdigkeitserkennung bekannt.

Ferner gibt es Bestrebungen, erweiterte Vitalparameter eines Fahrzeugführers zu erfassen. Dies ermöglicht es, den Fitness- oder Gesundheitszustand des Fahrzeugführers zu bestimmen. Informationen zum Gesundheitszustand können von Fahrerassistenzsystemen genutzt werden, um in Notsituationen einen Notstopp des Kraftfahrzeugs zu veranlassen. Dadurch werden sowohl der Fahrzeugführer und weitere Insassen als auch unbeteiligte potentielle Unfallopfer geschützt und schwere Unfälle vermieden.

Aus dem Stand der Technik sind kapazitive Verfahren zur Messung der Herzaktivität bekannt, die die Sitzbelegung über eine Kapazitätsänderung der Sitzheizungselektronik und eine durch den Herzschlag induzierte Bewegung des Brustkorbs auslesen können. Diese Verfahren sind jedoch sehr ungenau. Weiter bekannte, jedoch ebenfalls ungenaue Verfahren benutzen kamera-, UWB- oder radarbasierte Messungen.

Eine medizinische Notfallerkennung, zum Beispiel eines Herzinfarkts, ist derzeit nur über eine direkte Ableitung der Herzmuskelsignale möglich. Diese erfolgt in der Regel über Elektroden mit direktem Hautkontakt und ist daher für eine komfortable Integration in einem Kraftfahrzeug ungeeignet.

Aus der US 8,706,204 B2 ist ein System zur Beobachtung der Herzfrequenz eines Passagiers bekannt. Das System umfasst eine Vielzahl von verschiedenen Typen von Herzfrequenzsensoren, die auf einem Sitzkissen oder einer Sitzrückenlehne angeordnet sind. Die Herzfrequenzsensoren sind als Elektrokardiogrammsensoren ausgebildet. US2009/326399 offenbart ein Fahrzeug mit Gradiometer zur Erfassung von Magnetkardiogrammsignalen.

Aus der US 10,210,409 B1 ist ein System für eine kontaktlose Erfassung eines elektrodermalen Potentials bekannt, das in ein Kraftfahrzeug integriert ist. Aus dem elektrodermalen Potential kann ein Wach- oder Schlafzustand eines Fahrzeuginsassen ermittelt werden.

### Offenbarung

Der vorliegenden Erfindung liegt die Aufgabe zugrunde, ein Verfahren zur Bestimmung der Vitalfunktionen eines Fahrzeuginsassen bereitzustellen, welches präzise Messungen ermöglicht, und ohne Beeinträchtigung des Fahrzeuginsassen einfach anzuwenden ist.

Zur Lösung der der Erfindung zugrundeliegenden Aufgabe wird ein Verfahren zur Bestimmung der Vitalfunktionen eines Fahrzeuginsassen vorgeschlagen, wobei von einer in einen Fahrzeugsitz eines Kraftfahrzeugs integrierten Sensorvorrichtung berührungslos Messsignale eines Fahrzeuginsassen ermittelt werden, wobei aus den Messsignalen Kardiogrammsignale ermittelt werden, wobei eine Auswerteeinheit aus den Kardiogrammsignalen Vitalfunktionen des Fahrzeuginsassen ermittelt, wobei ferner vorgesehen ist, dass die Sensorvorrichtung eine Magnetfeldsensorvorrichtung ist, und dass die Kardiogrammsignale Magnetkardiogrammsignale sind.

Aus den Magnetkardiogrammsignalen kann die Auswerteeinheit Rückschlüsse auf den Fitness- und Gesundheitszustand des Fahrzeuginsassen ziehen. Insbesondere ermöglicht es die Auswertung der Magnetkardiogrammsignale, medizinisch relevante Notfälle am Herzen frühzeitig zu erkennen und einen sicheren Fahrzeugstopp herbeizuführen, bevor der Fahrzeuginsasse die Kontrolle über das Fahrzeug verliert und sich und andere gefährdet.

Gemäß der Erfindung ist vorgesehen, dass die Magnetfeldsensorvorrichtung ein Gradiometer mit mindestens zwei an zueinander beabstandeten Positionen angeordneten Magnetfeldsensoren ist, wobei die mindestens zwei Magnetfeldsensoren ein Magnetfeld an den beabstandeten Positionen messen und das Messsignal erzeugen, wobei das Magnetkardiogrammsignal weiter bevorzugt als Differenzsignal der Messsignale der mindestens zwei Magnetfeldsensoren ermittelt wird.

Die Magnetfelder, welche durch den Herzmuskel generiert werden, weisen eine Stärke im Bereich von 100 pT auf. Diese Magnetfeldstärken liegen deutlich unterhalb von typischen Magnetfeldstärken der Umgebung. So beträgt die Erdmagnetfeldstärke ca. 50 µT und die Magnetfelder im Kraftfahrzeug liegen im Bereich von 1 bis 10 nT. Durch die Ausgestaltung der Magnetfeldsensorvorrichtung als ein Gradiometer mit zwei an zueinander beabstandeten Positionen angeordneten Magnetfeldsensoren können diese Störfelder aus der Umgebung eliminiert werden. Hierfür wird das Magnetfeld gleichzeitig mit zwei Magnetfeldsensoren gemessen. Die Störfelder aus der Umgebung, welche an den beiden Positionen der Magnetfeldsensoren die gleiche Feldstärke aufweisen, können durch die Differenzbildung der Messsignale der mindestens zwei Magnetfeldsensoren eliminiert werden. Im Differenzsignal bleibt nur das von dem Herzen erzeugte Magnetfeld in Form des Magnetkardiogrammsignals übrig, da das von dem Herzen erzeugte Magnetfeld einen hohen Gradienten zwischen den beiden Positionen der Magnetfeldsensoren aufweist.

Das Differenzsignal kann dabei direkt von der Magnetfeldsensorvorrichtung erzeugt werden. Es ist aber auch möglich, dass das Differenzsignal in der Auswerteeinheit erzeugt wird, wobei in diesem Fall die Messsignale der Magnetfeldsensoren an die Auswerteeinheit übermittelt werden.

Bevorzugt ist vorgesehen, dass die mindestens zwei Magnetfeldsensoren einen Abstand zueinander von 0,5 cm bis 2 cm, weiter bevorzugt von 1 cm bis 1,5 cm, aufweisen.

Untersuchungen der Anmelderin haben gezeigt, dass bei einem Abstand zwischen 0,5 cm bis 2 cm die Biomagnetfelder, wie beispielsweise die von einem Herzen erzeugten Magnetfelder, ideal gemessen werden können.

Mit weiterem Vorteil kann vorgesehen sein, dass mehrere Magnetfeldsensorvorrichtungen vorgesehen sind, und/oder dass die Magnetfeldsensorvorrichtung im Fahrzeugsitz automatisch in der Nähe des Herzens des Fahrzeuginsassen positioniert wird.

Da es eine sehr große Streuung bzw. Varianz in der Größe möglicher Fahrzeuginsassen geben kann, ist es vorteilhaft, wenn mehrere Magnetfeldsensorvorrichtungen an verschiedenen Positionen in dem Fahrzeugsitz integriert sind. Alternativ oder zusätzlich kann vorgesehen sein, dass die Magnetfeldsensorvorrichtung aktiv im Fahrzeugsitz positioniert wird, sodass die Magnetfeldsensorvorrichtung unabhängig von der Größe des Fahrzeuginsassen immer in nächster Nähe zum Herzen positioniert wird. Hierdurch kann sichergestellt werden, dass ein ausreichend starkes Magnetkardiogrammsignal ermittelt werden kann.

Weiter bevorzugt ist vorgesehen, dass die Magnetfeldsensorvorrichtung in einer Rückenlehne des Fahrzeugsitzes angeordnet ist.

Gemäß der Erfindung ist vorgesehen, dass die Magnetfeldsensoren Stickstoff-Fehlstellensensoren sind, wobei jeder Stickstoff-Fehlstellensensor bevorzugt einen Diamanten, optische Filter und Fotodetektoren, und weiter bevorzugt einen Mikrowellenresonator und/oder eine Lichtquelle, insbesondere einen Laser, umfasst.

Der Mikrowellenresonator und/oder die Lichtquelle können jedoch auch beanstandet von der Magnetfeldsensorvorrichtung oder von den Magnetfeldsensoren angeordnet sein.

Grundsätzlich ist es möglich, dass zur Messung der Magnetfelder für die Ermittlung der Magnetkardiogrammsignale TMR, GMR oder Hall-Sensoren, SQUID-Sensoren oder Dampfzellen-Magnetometer verwendet werden. Derartige Sensoren haben aber in der Regel keine ausreichend hohe Empfindlichkeit. Hochempfindliche, supraleitende SQUID-Sensoren haben zwar die ausreichende Genauigkeit, benötigen jedoch eine aktive Kühlung mit flüssigem Stickstoff oder Helium und sind daher für den Einsatz in einem Fahrzeugsitz weniger geeignet. Dampfzellen-Magnetometer weisen zwar die benötigte Empfindlichkeit auf, haben jedoch nur einen begrenzten Dynamikbereich.

Gemäß der Erfindung ist daher vorgesehen, dass die Magnetfeldsensoren Stickstoff-Fehlstellensensoren sind. Stickstoff-Fehlstellensensoren beruhen auf der Messung eines Fluoreszenzspektrums von Stickstoffzentren in einem Diamanten. Das Spektrum eines Diamanten mit Stickstoff-Fehlstellen zeigt bei optischer Anregung eine Fluoreszenz im roten Wellenlängenbereich. Strahlt man neben der optischen Anregung Mikrowellenstrahlung ein, kommt es bei 2,88 GHz zu einem Einbruch der Fluoreszenz, da die Elektronen in diesem Fall vom mₛ= 0 Niveau des 3A-Zustandes auf das mₛ= +/-1 Niveau des 3E-Zustands gehoben werden und von dort nichtstrahlend rekombinieren. Bei einem externen Magnetfeld kommt es zur Aufspaltung der mₛ-Niveaus, das sogenannte Zeeman-Splitting, und es zeigen sich bei Auftragung der Fluoreszenz über die Frequenz der Mikrowellenanregung zwei Dips im Fluoreszenzspektrum, deren Frequenzabstand proportional zur magnetischen Feldstärke ist. Die Magnetfeldsensitivität wird dabei durch die minimal auflösbare Frequenzverschiebung definiert und kann bis 1 pT erreichen. Da das Stickstoff-Fehlstellenzentrum im einkristallinen Diamanten vier Möglichkeiten besitzt, sich im Kristallgitter anzuordnen, kommt es bei Anwesenheit eines gerichteten Magnetfeldes dazu, dass die im Kristall vorhandenen Stickstoff-Fehlstellenzentren je nach Lage im Kristall unterschiedlich stark auf das äußere Magnetfeld reagieren. Dadurch können im Maximalfall vier einander zugehörige Paare von Fluoreszenzminima im Spektrum auftauchen, aus deren Form und Lage zueinander Betrag und Richtung des Magnetfeldes eindeutig bestimmbar sind.

Der Aufbau und die Funktionsweise eines Stickstoff-Fehlstellensensors sind dem Fachmann darüber hinaus bekannt.

Mit weiterem Vorteil kann vorgesehen sein, dass die Magnetkardiogrammsignale von der Auswerteeinheit mit einem Hochpass und/oder Tiefpass gefiltert werden, und/oder dass die Auswerteeinheit eine Bias-Drift der Magnetfeldsensoren, insbesondere durch eine Mittelung der Magnetkardiogrammsignale über einen Zeitraum, welcher größer als die Herzrate ist, ermittelt.

Durch die Filterung mittels eines Tiefpasses werden Rauschanteile mit Frequenzen deutlich oberhalb der Herzfrequenz eliminiert. Die Bias-Drift kann ferner von dem hochpass- und/oder tiefpassgefilterten Signal abgezogen werden, sodass ein sauberes Magnetkardiogrammsignal vorliegt.

Ferner bevorzugt ist vorgesehen, dass die Auswerteeinheit aus den Magnetkardiogrammsignalen Vitalparameter, bevorzugt eine Herzrate, und/oder eine Herzratenvariabilität, und/oder eine Dauer und/oder Amplitude von EKG-äquivalenten Signalveränderungen, bevorzugt P-Welle, QRS-Komplex, T-Welle und entsprechender Kombinationen, ermittelt.

Mit weiterem Vorteil kann vorgesehen sein, dass die Auswerteeinheit auf Basis der Vitalparameter die Vitalfunktionen des Fahrzeuginsassen bestimmt, wobei die Auswerteeinheit bevorzugt eine Beurteilung erstellt, ob der Fahrzeuginsasse müde oder wach ist, unter Stress steht oder entspannt ist, ob eine akute oder chronische Anomalie der Herzfunktion vorliegt oder bevorsteht, oder ob Grunderkrankungen mit Beeinträchtigung der Herzfunktion vorliegen.

Für die Bestimmung der Vitalfunktionen des Fahrzeuginsassen auf Basis der Vitalparameter können verschiedene Verfahren eingesetzt werden. So ist es möglich, ein Schwellwertverfahren auf Basis einer vorgegebenen Tabelle mit entsprechenden Normwerten einzusetzen, wobei die Normwerte einer klinischen Diagnostik entnommen werden können. Ferner ist eine datengetriebene Auswertung beziehungsweise Interpretation mit Hilfe eines statistischen Klassifikationsverfahrens möglich. Hierbei kann ein Entscheidungsbaum-Verfahren oder ein Random Forest-Verfahren Einsatz finden. Eine weitere Möglichkeit besteht darin, eine Auswertung unter Verwendung tiefer neuronaler Netze einzusetzen.

Bevorzugt ist vorgesehen, dass auf Basis der Beurteilung weitere Maßnahmen getroffen werden, wobei die weiteren Maßnahmen einen Not-Stopp des Kraftfahrzeugs und/oder das Absetzen eines Notrufs und/oder die Übermittlung der Vitalfunktionen an medizinisches Personal umfassen.

Zusätzlich oder alternativ kann der Fahrer aktiv gewarnt werden, dass ein medizinisches Problem erkannt wurde. Wird von der Auswerteeinheit ein Notruf abgesetzt, so können gleichzeitig mit dem Absetzen des Notrufes die Vitalfunktionen, die Vitalparameter, die Magnetkardiogrammsignale oder die Beurteilung an das medizinische Personal mitgesendet werden, wodurch eine optimale Behandlung des Fahrzeuginsassen ohne weitere Verzögerung durch eine EKG-Messung vor Ort ermöglicht wird.

Ferner können für die Erstellung der Beurteilung weitere Informationen, wie beispielsweise das Alter oder das Gewicht des Fahrzeuginsassen herangezogen werden. Die weiteren Informationen können über Schnittstellen, wie beispielsweise über ein Multimedia- oder Infotainmentsystem oder über eine App, herangezogen werden.

Ferner kann bevorzugt vorgesehen sein, dass die Auswerteeinheit die Vitalfunktionen und/oder die Beurteilung an weitere Kraftfahrzeugeinrichtungen, insbesondere an ein Infotainmentsystem, an ein Fahrerassistenzsystem oder an ein Komfortsystem, sendet.

So können beispielsweise, wenn die Vitalfunktion und/oder die Beurteilung an ein Komfortsystem weitergeleitet werden, eine Massagefunktion, eine Beduftung des Fahrzeuginnenraums oder eine Ambientebeleuchtung aktiviert werden. Ferner können die Vitalfunktionen und die Beurteilung zur Verbesserung der Müdigkeitserkennung beitragen und somit die Genauigkeit einer Pausenempfehlung verbessern. Hierfür kann das Verfahren mit der Auswertung von Daten einer Innenraumkamera kombiniert werden.

Die Beurteilung kann mit einem Konfidenzwert versehen werden, der die statistische Sicherheit des Klassifikationsergebnisses bzw. der Beurteilung widerspiegelt.

Von Vorteil sind eine Einrichtung, eingerichtet zur Bestimmung der Vitalfunktionen eines Fahrzeuginsassen wie vorstehend beschrieben, umfassend eine Sensorvorrichtung, die zur Installation in einem Fahrzeugsitz eines Fahrzeugs, insbesondere Kraftfahrzeugs ausgebildet und dazu eingerichtet ist, Messsignale eines Fahrzeuginsassen zu ermitteln, wobei die Sensorvorrichtung als Magnetfeldsensorvorrichtung ausgeführt ist, und weiter umfassend eine mit der Magnetfeldsensorvorrichtung kommunikations-verbundene Auswerteeinheit, welche dazu ausgebildet ist, aus aus den Messsignalen erhaltenen Kardiogrammsignalen Vitalfunktionen eines Fahrzeuginsassen zu ermitteln, wobei die Kardiogrammsignale Magnetkardiogrammsignale sind.

In vorteilhafter Ausgestaltung kann dabei vorgesehen sein, dass die Sensorvorrichtung oder auch die Sensorvorrichtung und die Auswerteeinheit im Fahrzeugsitz angeordnet sind.

In der Fertigung kann es ohne weiteres ersichtlich von Vorteil sein, die Sensorvorrichtung oder die Sensorvorrichtung Auswerteeinrichtung bereits im Rahmen der Herstellung des Fahrzeugsitzes in denselben zu integrieren.

Die Auswertevorrichtung kann in vorteilhafter Weise in Form eines Software-Bausteins in einem Steuergerät bzw. Computer implementiert werden. Somit ist es möglich, die Auswertevorrichtung auch in ein im Fahrzeug ohnehin vorhandenes Steuergerät oder einen Computer, beispielsweise in einen Infotainment-Computer oder einen Zentral-Computer zu integrieren, welcher mit der Sensorvorrichtung über diskrete Leitungen, einen drahtgebunenen Datenbus oder über eine Funkschnittstelle verbunden sein kann. Eine weitere Lösung der zugrundeliegenden Aufgabe besteht in der Bereitstellung eines Kraftfahrzeuges umfassend einen Fahrzeugsitz und eine Auswerteeinheit ausgebildet zur Durchführung eines vorbeschriebenen Verfahrens.

Sämtliche im Zusammenhang mit dem vorbeschriebenen Verfahren erläuterten Funktionen, Merkmale und Ausgestaltungen können in entsprechender Art und Weise auch auf das Kraftfahrzeug übertragen werden.

Die Beschreibung wird nachstehend näher anhand der beigefügten Figuren erläutert.

Es zeigen
- Fig. 1: einen Fahrzeugsitz mit einer Magnetfeldsensorvorrichtung,
- Fig. 2: eine Magnetfeldsensorvorrichtung, und
- Fig. 3: ein Ablaufdiagramm des Verfahrens..

Ein Verfahren 100 zur Bestimmung der Vitalfunktionen eines Fahrzeuginsassen 10 wird nachstehend anhand der Figuren näher erläutert.

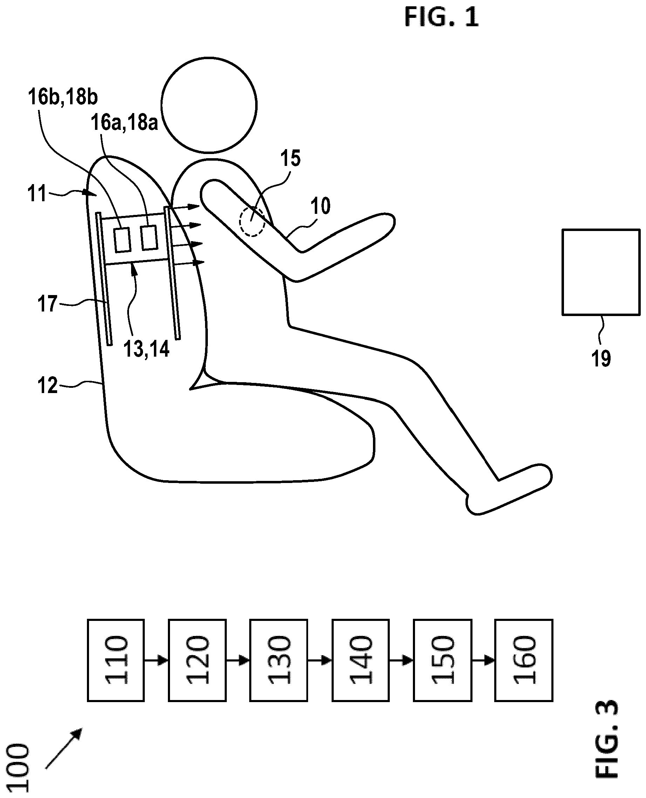

Verfahrensgemäß ist ein in Fig. 1 dargestellter Fahrzeugsitz 11 eines nicht näher gezeigten Kraftfahrzeugs vorgesehen. In dem Fahrzeugsitz 11, insbesondere in der Rückenlehne 12, ist eine Sensorvorrichtung 13 angeordnet. Die Sensorvorrichtung 13 ist dabei als Magnetfeldsensorvorrichtung 14 ausgebildet und ermittelt Messsignale eines Herzens 15 des Fahrzeuginsassen 10 (Fig. 3, Schritt 110). Die Magnetfeldsensorvorrichtung 14 umfasst zwei an zueinander beabstandeten Positionen angeordnete Magnetfeldsensoren 16a, 16b. Dabei ist ein erster Magnetfeldsensor 16a näher am Herzen 15 des Fahrzeuginsassen 10 angeordnet als ein zweiter Magnetfeldsensor 16b. Die beiden Magnetfeldsensoren 16a, 16b messen somit das Magnetfeld an den beabstandeten Positionen und erzeugen jeweils die Messsignale. Die von den Magnetfeldsensoren 16a, 16b ermittelten Messsignale werden funk- oder kabelbasiert an eine Auswerteeinheit 19 übermittelt (Fig. 3, Schritt 120). Durch Differenzbildung der Messsignale der beiden Magnetfeldsensoren 16a, 16b können Störfelder, wie beispielsweise das natürlich Erdmagnetfeld oder im Kraftfahrzeug auftretende Magnetfelder, eliminiert werden. Das Differenzsignal der Messsignale ist ein Magnetkardiogrammsignal. Die Auswerteeinheit 19 ermittelt aus den Magnetkardiogrammsignalen Vitalparameter (Fig. 3, Schritt 130). Hierfür werden die Magnetkardiogrammsignale zunächst mit einem Tiefpass gefiltert. Ferner wird eine Bias-Drift der Magnetfeldsensoren 16a, 16b durch eine Mittelung der Magnetkardiogrammsignale über einen Zeitraum, welcher größer als die Herzrate ist, ermittelt. Die Bias-Drift der Magnetfeldsensoren 16a, 16b wird von den Magnetkardiogrammsignalen subtrahiert. Aus den Vitalparametern, welche beispielsweise eine Herzrate und/oder eine Herzratenvariabilität sein können, ermittelt die Auswerteeinheit 19 die Vitalfunktionen des Fahrzeuginsassen 10 und erstellt eine Beurteilung eines aktuellen Zustandes bzw. einer psychischen oder physischen Verfassung des Fahrzeuginsassen (Schritt 140). Dieser Zustand kann beispielsweise eine Beurteilung umfassen, ob der Fahrzeuginsasse 10 müde oder wach ist, unter Stress steht oder entspannt ist, ob eine akute oder chronische Anomalie der Herzfunktion vorliegt oder bevorsteht oder ob Grunderkrankungen mit Beeinträchtigung der Herzfunktion vorliegen. Stellt die Auswerteeinheit 19 fest, dass eine die Fahrtüchtigkeit des Fahrzeuginsassen 10 beeinträchtigende Vitalfunktion vorliegt, so sendet die Auswerteeinheit 19 die Vitalfunktionen und/oder die Beurteilung an weitere Kraftfahrzeugeinrichtungen, wie beispielsweise an ein Fahrerassistenzsystem (Schritt 150). Das Fahrerassistenzsystem ist vorzugsweise dazu ausgebildet, in Abhängigkeit der von der Auswerteeinheit erhaltenen Informationen über die Verfassung des Fahrzeuginsassen weitergehende Maßnahmen auszuführen, beispielsweise einen automatischen Notruf (e-Call) an eine Leitzentrale abzusetzen, das Fahrzeug kontrolliert zum Stillstand zu bringen oder andere je nach Zustand des Fahrzeuginsassen hilfreiche oder sinnvolle Maßnahmen einzuleiten (Schritt 160). Die beiden Magnetfeldsensoren 16a, 16b weisen einen Abstand von 0,5 cm bis 2 cm zueinander auf. Die Magnetfeldsensorvorrichtung 14 ist als ein Gradiometer ausgebildet und mittels eines Führungssystems 17 in der Rückenlehne 12 in der Höhe verstellbar, sodass die Magnetfeldsensorvorrichtung 14 stets in der Nähe des Herzens 15 des Fahrzeuginsassen 10 positioniert werden kann. Die beiden Magnetfeldsensoren 16a, 16b sind gemäß der Erfindung als Stickstoff-Fehlstellensensoren 18a, 18b ausgebildet und weisen nicht näher dargestellt einen Diamanten, optische Filter und Photodetektoren auf.

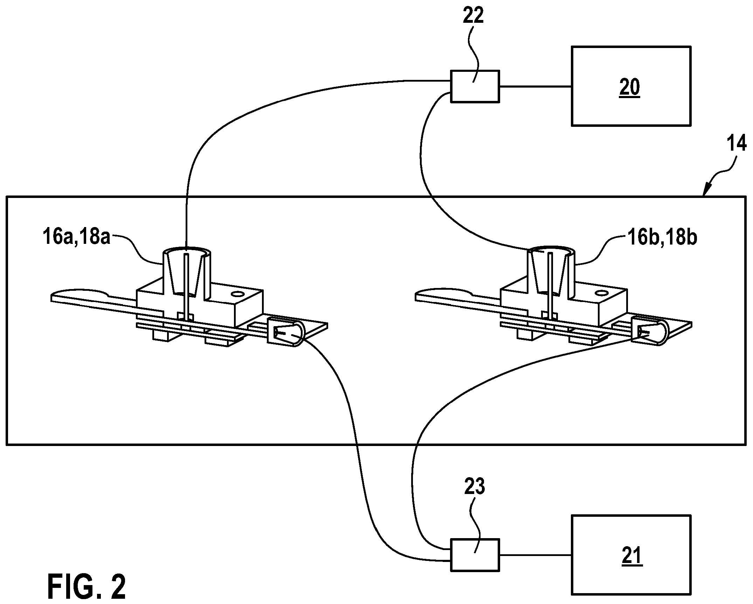

Fig. 2 zeigt eine Detailansicht der Magnetfeldsensorvorrichtung 14 der Fig. 1. Die Magnetfeldsensorvorrichtung 14 umfasst zwei Magnetfeldsensoren 16a, 16b, welche gemäß der Erfindung als Stickstoff-Fehlstellensensoren 18a, 18b ausgebildet sind. Die beiden Magnetfeldsensoren 16a, 16b sind in einem Abstand von 0,5 cm bis 2 cm zueinander angeordnet und umfassen jeweils einen stickstofffehlstellendotierten Diamanten, optische Filter und Photodetektoren. Die Magnetfeldsensorvorrichtung 14 umfasst ferner einen Laser 20 und einen Mikrowellenresonator 21. Die beiden Stickstoff-Fehlstellensensoren 18a, 18b werden von dem Laser 20 mit Licht beaufschlagt, wobei ein Fasersplitter 22 im Strahlengang vorgesehen ist. Ferner wird von dem Mikrowellenresonator 21 Mikrowellenstrahlung auf die Stickstoff-Fehlstellensensoren 18a, 18b eingestrahlt, wobei im Strahlengang ein Mikrowellensplitter 23 angeordnet ist.

## Patentansprüche

1. Verfahren (100) zur Bestimmung der Vitalfunktionen eines Fahrzeuginsassen (10), wobei von einer in einen Fahrzeugsitz (11) eines Kraftfahrzeugs integrierten Sensorvorrichtung (13) Messsignale eines Fahrzeuginsassen (10) ermittelt werden, wobei aus den Messsignalen Kardiogrammsignale ermittelt werden, wobei eine Auswerteeinheit (19) aus den Kardiogrammsignalen Vitalfunktionen des Fahrzeuginsassen (10) ermittelt, wobei die Sensorvorrichtung (13) eine Magnetfeldsensorvorrichtung (14) ist und die Kardiogrammsignale Magnetkardiogrammsignale sind, wobei die Magnetfeldsensorvorrichtung (14) ein Gradiometer mit mindestens zwei an zueinander beabstandeten Positionen angeordneten Magnetfeldsensoren (16a, 16b) ist, wobei die mindestens zwei Magnetfeldsensoren (16a, 16b) ein Magnetfeld an den beabstandeten Positionen messen und die Messsignale erzeugen und wobei das Kardiogrammsignal bevorzugt als Differenzsignal der Messsignale der mindestens zwei Magnetfeldsensoren (16a, 16b) ermittelt wird,
**dadurch gekennzeichnet**
**dass** die Magnetfeldsensoren (16a, 16b) Stickstoff-Fehlstellensensoren (18a, 18b) sind, wobei jeder Stickstoff-Fehlstellensensor (18a, 18b) bevorzugt einen Diamanten, optische Filter und Fotodetektoren, und weiter bevorzugt einen Mikrowellenresonator (21) und/oder eine Lichtquelle, insbesondere einen Laser (20), umfasst..

2. Verfahren (100) nach Anspruch 1 , **dadurch gekennzeichnet, dass** mehrere Magnetfeldsensorvorrichtungen (14) vorgesehen sind, und/oder dass die Magnetfeldsensorvorrichtung (14) im Fahrzeugsitz (11) automatisch in der Nähe des Herzens (15) des Fahrzeuginsassen (10) positioniert wird.

3. Verfahren (100) nach einem der vorgenannten Ansprüche, **dadurch gekennzeichnet, dass** die Magnetkardiogrammsignale von der Auswerteeinheit (19) mit einem Hochpass und/oder Tiefpass gefiltert werden, und/oder dass die Auswerteeinheit (19) eine Bias-Drift der Magnetfeldsensoren (16a, 16b), insbesondere durch eine Mittelung der Magnetkardiogrammsignale über eine Zeitraum, welcher größer als die Herzrate ist, ermittelt.

4. Verfahren (100) nach einem der vorgenannten Ansprüche, **dadurch gekennzeichnet, dass** die Auswerteeinheit (19) aus den Magnetkardiogrammsignalen Vitalparameter, bevorzugt eine Herzrate, und/oder eine Herzratenvariabilität, und/oder eine Dauer und/oder Amplitude von EKG-äquivalenten Signalveränderungen, bevorzugt P-Welle, QRS-Komplex, T-Welle und entsprechender Kombinationen, ermittelt.

5. Verfahren (100) nach Anspruch 4, **dadurch gekennzeichnet, dass** die Auswerteeinheit (19) auf Basis der Vitalparameter die Vitalfunktionen des Fahrzeuginsassen (10), bevorzugt mittels eines statistischen Klassifikationsverfahrens, bestimmt, wobei die Auswerteeinheit (19) weiter bevorzugt eine Beurteilung erstellt, ob der Fahrzeuginsasse (10) müde oder wach ist, unter Stress steht oder entspannt ist, ob eine akute oder chronische Anomalie der Herzfunktion vorliegt oder bevorsteht, oder ob Grunderkrankungen mit Beeinträchtigung der Herzfunktion vorliegen.

6. Verfahren (100) nach Anspruch 5, **dadurch gekennzeichnet, dass** auf Basis der Beurteilung weitere Maßnahmen getroffen werden, wobei die weiteren Maßnahmen einen Not-Stopp des Kraftfahrzeugs und/oder das Absetzen eines Notrufs und/oder die Übermittlung der Vitalfunktionen an medizinisches Personal umfassen.

7. Verfahren (100) nach einem der vorgenannten Ansprüche, **dadurch gekennzeichnet, dass** die Auswerteeinheit (19) die Vitalfunktionen und/oder die Beurteilung an weitere Kraftfahrzeugeinrichtungen, insbesondere an ein Infotainmentsystem, an ein Fahrerassistenzsystem oder an ein Komfortsystem, sendet.

8. Einrichtung, eingerichtet zur Bestimmung der Vitalfunktionen eines Fahrzeuginsassen (10) nach einem der Ansprüche 1 bis 7,
umfassend eine Sensorvorrichtung (13), die Installation in einem Fahrzeugsitz (11) eines Fahrzeugs, insbesondere Kraftfahrzeugs ausgebildet und dazu eingerichtet ist, Messsignale eines Fahrzeuginsassen (10) zu ermitteln,
wobei die Sensorvorrichtung (13) als Magnetfeldsensorvorrichtung (14) ausgeführt ist, und
weiter umfassend eine Auswerteeinheit (19), welche dazu ausgebildet ist, aus aus den Messsignalen erhaltenen Kardiogrammsignalen Vitalfunktionen eines Fahrzeuginsassen (10) zu ermitteln,
wobei die Kardiogrammsignale Magnetkardiogrammsignale sind und wobei die Magnetfeldsensorvorrichtung (14) ein Gradiometer mit mindestens zwei an zueinander beabstandeten Positionen angeordneten Magnetfeldsensoren (16a, 16b) ist, wobei die mindestens zwei Magnetfeldsensoren (16a, 16b) ausgebildet sind, ein Magnetfeld an den beabstandeten Positionen zu messen und die Messsignale zu erzeugen und wobei die Auswerteeinheit (19) eingerichtet ist, das Kardiogrammsignal bevorzugt als Differenzsignal der Messsignale der mindestens zwei Magnetfeldsensoren (16a, 16b) zu ermitteln,
**dadurch gekennzeichnet dass**
die Magnetfeldsensoren (16a, 16b) Stickstoff-Fehlstellensensoren (18a, 18b) sind, wobei jeder Stickstoff-Fehlstellensensor (18a, 18b) bevorzugt einen Diamanten, optische Filter und Fotodetektoren, und weiter bevorzugt einen Mikrowellenresonator (21) und/oder eine Lichtquelle, insbesondere einen Laser (20), umfasst.

9. Einrichtung nach Anspruch 8, **dadurch gekennzeichnet, dass** die Sensorvorrichtung (13) oder die Sensorvorrichtung (13) und die Auswerteeinheit (19) im Fahrzeugsitz angeordnet sind.

10. Kraftfahrzeug umfassend den Fahrzeugsitz (11) und die Einrichtung nach Anspruch 9.

## Claims

1. Method (100) for determining the vital functions of a vehicle occupant (10), a sensor device (13) integrated in a vehicle seat (11) of a motor vehicle identifying measurement signals relating to a vehicle occupant (10), the measurement signals being used to identify cardiogram signals, an evaluation unit (19) using the cardiogram signals to identify vital functions of the vehicle occupant (10), the sensor device (13) being a magnetic field sensor device (14) and the cardiogram signals being magnetic cardiogram signals, the magnetic field sensor device (14) being a gradiometer having at least two magnetic field sensors (16a, 16b) arranged at spaced-apart positions, the at least two magnetic field sensors (16a, 16b) measuring a magnetic field at the spaced positions and generating the measurement signals, and the cardiogram signal being identified preferably as a difference signal relating to the measurement signals of the at least two magnetic field sensors (16a, 16b),
**characterized**
**in that** the magnetic field sensors (16a, 16b) are nitrogen-vacancy defect sensors (18a, 18b), each nitrogen-vacancy defect sensor (18a, 18b) preferably comprising a diamond, optical filters and photodetectors, and more preferably a microwave resonator (21) and/or a light source, in particular a laser (20).

2. Method (100) according to Claim 1, **characterized in that** there is provision for multiple magnetic field sensor devices (14), and/or **in that** the magnetic field sensor device (14) in the vehicle seat (11) is automatically positioned near the heart (15) of the vehicle occupant (10).

3. Method (100) according to either of the preceding claims, **characterized in that** the magnetic cardiogram signals are filtered by the evaluation unit (19) using a high-pass and/or low-pass filter, and/or **in that** the evaluation unit (19) identifies a bias drift of the magnetic field sensors (16a, 16b), in particular by averaging the magnetic cardiogram signals over a period greater than the heart rate.

4. Method (100) according to one of the preceding claims, **characterized in that** the evaluation unit (19) uses the magnetic cardiogram signals to identify vital parameters, preferably a heart rate, and/or a heart rate variability, and/or a duration and/or amplitude of ECG-equivalent signal changes, preferably P-wave, QRS complex, T-wave and relevant combinations.

5. Method (100) according to Claim 4, **characterized in that** the evaluation unit (19) takes the vital parameters as a basis for determining the vital functions of the vehicle occupant (10), preferably by means of a statistical classification method, the evaluation unit (19) more preferably producing an assessment of whether the vehicle occupant (10) is tired or awake, is under stress or is relaxed, whether an acute or chronic abnormality in cardiac function exists or is imminent, or whether underlying diseases that impair cardiac function exist.

6. Method (100) according to Claim 5, **characterized in that** the assessment is taken as a basis for taking further measures, the further measures including an emergency stop by the motor vehicle and/or placing an emergency call and/or transmitting the vital functions to medical personnel.

7. Method (100) according to one of the preceding claims, **characterized in that** the evaluation unit (19) sends the vital functions and/or the assessment to other motor vehicle apparatuses, in particular to an infotainment system, to a driver assistance system or to a comfort system.

8. Apparatus configured to determine the vital functions of a vehicle occupant (10) according to one of Claims 1 to 7,
comprising a sensor device (13) designed for installation in a vehicle seat (11) of a vehicle, in particular a motor vehicle, and configured to identify measurement signals relating to a vehicle occupant (10),
the sensor device (13) being a magnetic field sensor device (14), and
further comprising an evaluation unit (19) designed to use cardiogram signals obtained from the measurement signals to identify vital functions of a vehicle occupant (10),
the cardiogram signals being magnetic cardiogram signals and the magnetic field sensor device (14) being a gradiometer having at least two magnetic field sensors (16a, 16b) arranged at spaced-apart positions, the at least two magnetic field sensors (16a, 16b) being designed to measure a magnetic field at the spaced positions and to generate the measurement signals, and the evaluation unit (19) being configured to identify the cardiogram signal preferably as a difference signal relating to the measurement signals of the at least two magnetic field sensors (16a, 16b),
**characterized in that**
the magnetic field sensors (16a, 16b) are nitrogen-vacancy defect sensors (18a, 18b), each nitrogen-vacancy defect sensor (18a, 18b) preferably comprising a diamond, optical filters and photodetectors, and more preferably a microwave resonator (21) and/or a light source, in particular a laser (20).

9. Apparatus according to Claim 8, **characterized in that** the sensor device (13) or the sensor device (13) and the evaluation unit (19) are arranged in the vehicle seat.

10. Motor vehicle comprising the vehicle seat (11) and the apparatus according to Claim 9.

## Revendications

1. Procédé (100) pour déterminer les fonctions vitales d'un occupant (10) d'un véhicule, des signaux de mesure d'un occupant (10) du véhicule étant déterminés par un dispositif de détection (13) intégré dans un siège (11) d'un véhicule à moteur, des signaux de cardiogramme étant déterminés à partir des signaux de mesure, une unité d'analyse (19) déterminant des fonctions vitales de l'occupant (10) du véhicule à partir des signaux de cardiogramme, le dispositif de détection (13) étant un dispositif de détection de champ magnétique (14) et les signaux de cardiogramme étant des signaux de magnétocardiogramme, le dispositif de détection de champ magnétique (14) étant un gradiomètre avec au moins deux capteurs de champ magnétique (16a, 16b) disposés sur des positions espacées l'une par rapport à l'autre, les au moins deux capteurs de champ magnétique (16a, 16b) mesurant un champ magnétique sur les positions espacées et générant les signaux de mesure, le signal de cardiogramme étant déterminé de préférence comme signal de différence des signaux de mesure des au moins deux capteurs de champ magnétique (16a, 16b),
**caractérisé en ce**
**que** les capteurs de champ magnétique (16a, 16b) sont des capteurs de défaut d'azote (18a, 18b), chaque capteur de défaut d'azote (18a, 18b) comprenant de manière préférée un diamant, des filtres optiques et des photodétecteurs, et de manière davantage préférée un résonateur hyperfréquence (21) et/ou une source de lumière, en particulier un laser (20).

2. Procédé (100) selon la revendication 1, **caractérisé en ce que** plusieurs dispositifs de détection de champ magnétique (14) sont prévus et/ou que le dispositif de détection de champ magnétique (14) est positionné automatiquement dans le siège (11) de véhicule à proximité du cœur (15) de l'occupant (10) du véhicule.

3. Procédé (100) selon l'une des revendications précédentes, **caractérisé en ce que** les signaux de magnétocardiogramme sont filtrés par l'unité d'analyse (19) avec un filtre passe-haut et/ou un filtre passe-bas et/ou que l'unité d'analyse (19) détermine une dérive de polarisation des capteurs de champ magnétique (16a, 16b), en particulier en calculant la moyenne des signaux de magnétocardiogramme sur une période de temps qui est supérieure à la fréquence cardiaque.

4. Procédé (100) selon l'une des revendications précédentes, **caractérisé en ce que** l'unité d'analyse (19) détermine à partir des signaux de magnétocardiogramme des paramètres vitaux, de manière préférée une fréquence cardiaque et/ou une variabilité de fréquence cardiaque et/ou une durée et/ou une amplitude de modifications de signal équivalentes à l'ECG, de manière préférée une onde P, un complexe QRS, une onde T et des combinaisons correspondantes.

5. Procédé (100) selon la revendication 4, **caractérisé en ce que** l'unité d'analyse (19) définit les fonctions vitales de l'occupant (10) du véhicule sur la base des paramètres vitaux, de manière préférée au moyen d'un procédé de classification statistique, l'unité d'analyse (19) produisant en outre de manière préférée une évaluant pour savoir si l'occupant (10) du véhicule est fatigué ou éveillé, stressé ou détendu, si une anomalie aiguë ou chronique de la fonction cardiaque est présente ou imminente, ou si des maladies de base avec altération de la fonction cardiaque sont présentes.

6. Procédé (100) selon la revendication 5, **caractérisé en ce que** d'autres mesures sont prises sur la base de l'évaluation, les autres mesures comprenant un arrêt d'urgence du véhicule à moteur et/ou l'émission d'un appel d'urgence et/ou la transmission des fonctions vitales au personnel médical.

7. Procédé (100) selon l'une des revendications précédentes, **caractérisé en ce que** l'unité d'analyse (19) envoie les fonctions vitales et/ou l'évaluation à d'autres équipements du véhicule à moteur, en particulier à un système d'infodivertissement, à un système d'assistance au conducteur ou à un système de confort.

8. Equipement mis au point pour déterminer les fonctions vitales d'un occupant (10) d'un véhicule selon l'une des revendications 1 à 7,
comprenant un dispositif de détection (13), l'installation dans un siège (11) d'un véhicule, en particulier d'un véhicule à moteur, étant formée et mise au point pour déterminer des signaux de mesure d'un occupant (10) du véhicule le dispositif de détection (13) étant réalisé sous la forme d'un dispositif de détection de champ magnétique (14), et
comprenant par ailleurs une unité d'analyse (19) qui est formée pour déterminer des fonctions vitales d'un occupant (10) d'un véhicule à partir des signaux de cardiogramme obtenus à partir des signaux de mesure,
les signaux de cardiogramme étant des signaux de magnétocardiogramme et le dispositif de détection de champ magnétique (14) étant un gradiomètre avec au moins deux capteurs de champ magnétique (16a, 16b) disposés sur des positions espacées l'une par rapport à l'autre, les au moins deux capteurs de champ magnétique (16a, 16b) étant formés pour mesurer un champ magnétique sur les positions espacées et pour générer les signaux de mesure, l'unité d'analyse (19) étant mise au point pour déterminer le signal de cardiogramme de manière préférée comme signal de différence des signaux de mesure des au moins deux capteurs de champ magnétique (16a, 16b), **caractérisé en ce que**
les capteurs de champ magnétique (16a, 16b) sont des capteurs de défaut d'azote (18a, 18b), chaque capteur de défaut d'azote (18a, 18b) comprenant de manière préférée un diamant, des filtres optiques et des photodétecteurs, et de manière davantage préférée un résonateur hyperfréquence (21) et/ou une source de lumière, en particulier un laser (20).

9. Equipement selon la revendication 8, **caractérisé en ce que** le dispositif de détection (13) ou le dispositif de détection (13) et l'unité d'analyse (19) sont disposés dans le siège du véhicule.

10. Véhicule à moteur comprenant le siège (11) de véhicule et l'équipement selon la revendication 9.
